# EUROPÄISCHE PATENTSCHRIFT

(11) **EP 1 305 031 B1**
(45) Veröffentlichungstag und Bekanntmachung des Hinweises auf die Patenterteilung: **14.05.2008**
(21) Anmeldenummer: 01945307.5
(22) Anmeldetag: 23.06.2001
(51) Int. Cl.: A61K 31/66, A61P 31/04, A61P 33/02, A61P 33/06

(54) **KOMBINATIONSPRÄPARATE VON 3-N-FORMYLHYDROXYLAMINOPROPYLPHOSPHONSÄUREDERIVATEN ODER 3-N-ACETYLHYDROXYLAMINOPROPYLPHOSPHONSÄUREDERIVATEN MIT SPEZIELLEN PHARMAZEUTISCHEN WIRKSTOFFEN**
COMBINATION PREPARATIONS OF 3-N-FORMYLHYDROXYLAMINOPROPYL PHOSPHONIC ACID DERIVATIVES OR 3-N-ACETYLHYDROXYLAMINOPROPYL PHOSPHONIC ACID DERIVATIVES COMBINED WITH SPECIFIC PHARMACEUTICAL ACTIVE AGENTS
PREPARATIONS DE DERIVES D'ACIDE 3-N-FORMYLHYDROXYLAMINOPROPYLPHOSPHONIQUE OU DE DERIVES D'ACIDE 3-N-ACETYLHYDROXYLAMINOPROPYLPHOSPHONIQUE COMBINES AVEC DES PRINCIPES ACTIFS PHARMACEUTIQUES SPECIAUX

(30) Priorität: 29.06.2000 DE 10030781
(43) Veröffentlichungstag der Anmeldung: 02.05.2003
(73) Patentinhaber: BioAgency AG, 22525 Hamburg (DE)
(72) Erfinder: JOMAA, Hassan, 35392 Giessen (DE); WIESNER, Jochen, 35394 Giessen (DE)
(74) Vertreter: Ahrens, Gabriele
(86) Internationale Anmeldenummer: PCT/EP2001/007140
(87) Internationale Veröffentlichungsnummer: WO 2002/000208

(56) Entgegenhaltungen:
- US-A- 4 330 529
- PATENT ABSTRACTS OF JAPAN vol. 0061, no. 18, 2. Juli 1982 (1982-07-02) & JP 57 046917 A (FUJISAWA PHARMACEUTICAL), 17. März 1982 (1982-03-17)
- DATABASE WPI Week 198521 Derwent Publications Ltd., London, GB; AN 1985-126148 XP002187684 FUJISAWA PHARMACEUTICAL: "Cephalexin-fosmidomycin salt-prepared from mixture of their solutions, has antibiotic and antimicrobial activity" & JP 60 064991 A (FUJISAWA PHARMACEUTICAL) , 13. April 1985 (1985-04-13)
- H.C.NEU, T.KAMIMURA: "Synergy of fosmidomycin (FR-31564) and other antimicrobial agents" ANTIMICROBIAL AGENTS AND CHEMOTHERAPY, Bd. 22, Nr. 4, 1982, Seiten 560-563, XP002113261
- Y.YOKOTA, T,MURAKAWA, M.NISHIDA: "In vitro synergism of FR-31564, a new phosphonic acid antibiotic" THE JOURNAL OF ANTIBIOTICS, Bd. 34, Nr. 7, 1981, Seiten 876-883, XP000867644

## Beschreibung

Die vorliegende Erfindung betrifft pharmazeutische Präparate, die als Wirkstoffe 3-N-Formylhydroxylaminopropylphosphonsäurederivate oder 3-N-Acetylhydroxylaminopropylphosphonsäurederivate enthalten, in Kombination mit speziellen pharmazeutischen Wirkstoffen.

Die Verwendung von 3-N-Formylhydroxylaminopropylphosphonsäurederivaten und 3-N-Acetylhydroxylaminopropylphosphonsäurederivaten zur prophylaktischen und therapeutischen Behandlung von infektiösen Prozessen, insbesondere Infektionen, die durch einzellige Parasiten (im Sinne dieser Erfindung : Protozoen) oder mehrzellige Parasiten hervorgerufen werden, sind bereits aus der DE-A1-198 25585 bekannt. Eine bakterielle Wirksamkeit ist bereits in der DE-A1-27 33 658 beschrieben. Auch wenn diese Verbindungen bei der Behandlung von Infektionen, die durch Parasiten oder Bakterien hervorgerufen werden, gute Ergebnisse zeigen, so zeigen auch diese Medikamente unerwünschte Nebenwirkungen.

Kombinationen von Fosmydomycin (FR 31564) und aktiviertem Fosmydomycin (FR 90098) mit Antibiotika sind bereits bekannt und zwar mit Tetracyclinen (JP 57 046917 A, Neu H. C.; Kamimura T.; Synergy of fosmydomycin (FR-31564) and other antimicrobial agents, Antimicrobial Agents and Chemotherapy (22), 560-563 (1982)), beta-Lactam-Antibiotika (US 4330529, Neu et al (supra)), insbesondere Cephalosporine (JP 60 064991 A), Aminoglycosid-Antibiotika (US 4330529) sowie Trimethoprim (Tetroxoprim) (Neu et al (supra)).

Die vorliegende Erfindung hat sich deshalb zur Aufgabe gestellt, die Wirkung dieser pharmazeutischen Mittel zu steigern, ohne dass dies mit einer Steigerung der Nebenwirkungen dieser Wirkstoffe einhergeht. Es sollen Arzneimittel zur Vefügung gestellt werden, die eine Verminderung der Nebenwirkungen bewirken. Ziel ist es dabei, die therapeutische Anwendungsbreite dieser pharmazeutischen Mittel zu vergrössern, insbesondere auch auf die Behandlung von problematischen Gruppen wie z. B. Kindern und schwangeren Frauen auszudehnen. Es soll dieantiparasitäre Wirkung verstärkt werden, so dass diese phramzeutischen Mittel in geringeren Dosen verabreicht werden können und somit eine Verminderung oder Aufhebung der durch diese Mittel hervorgerufenen Nebenwirkungen erfolgt.

Es wurde nun überraschenderweise gefunden, daß 3-N-Formylhydroxylaminopropylphosphonsäurederivate und 3-N-Acetylhydroxylaminopropylphosphonsäurederivate in Kombination mit einem weiteren pharmazeutischen Mittel, das aus der Gruppe ausgewählt ist, die aus Clindamycin, Lincomycin, Pirlimycin und andere Lincosamide, Azithromycin, Erythromycin, Spiramycin, Josamycin, Roxithromycin, Clarithromycin, Midecamycin Lumefantrin, Tafenoquin (WR238,605), Pyronaridin, Dihydroartemisinin, Artemether, Arteether und Artesunat, besteht, eine deutlich höhere therapeutische Effizienz als bei der Monotherapie zeigt. Diese Kombinationspräparate eignen sich insbesondere zur Behandlung von Malaria.

Unter 3-N-Formyl-hydroxylamino-propylphosphonsäurederivaten und 3-N-Acetyl-hydroxylamino-propylphosphonsäurederivaten sind erfindungsgemäß Verbindungen der Formel (I) 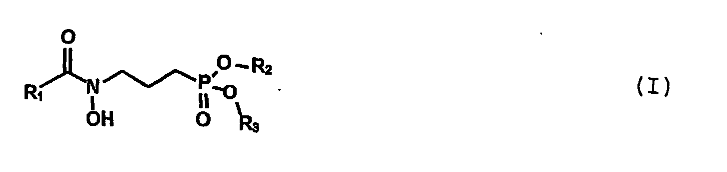 zu verstehen,
in der R₁ aus der Gruppe ausgewählt ist, die aus Wasserstoff und Methyl besteht,
und in der R₂ und R₃ unabhängig voneinander aus der Gruppe ausgewählt sind, die aus Wasserstoff, substituiertem oder unsubstituiertem Alkyl, substituiertem oder unsubstituiertem Acyl, substituiertem oder unsubstituiertem Aryl, substituiertem oder unsubstituiertem Aralkyl, substituiertem oder unsubstituiertem Cycloalkyl, einem substituiertem oder unsubstituiertem Silyl, substituiertem oder unsubstituiertem heterocyclischem Rest besteht, oder zusammen eine substituierte oder unsubstituierte C₁₋₅-Alkylkette bilden, wobei die Alkylguppen gesättigt oder eine oder mehrere Doppelbindungen oder Dreifachbindungen enthalten können.

Als zweites pharmazeutisches Mittel sind insbesondere für die Behandlung parasitärer Infektionen Lumefantrin, Tafenoquin (WR238,605), Pyronaridin, Dihydroartemisinin, Artemether, Arteether, Artesunat, Clindamycin und Azithromycin bevorzugt.

Für die Behandlung sowohl bakterieller als auch parasitärer Infektionen sind ganz besonders Clindamycin und Azithromycin bevorzugt. Eine besondere Eignung zeigt das Kombinationspräparat mit Clindamycin oder Azithromycin zur Behandlung von Infektionen durch Helicobacter pylori.

Unter Kombinationspräparat sind auch die jeweiligen Salze, wie zum Beispiel insbesondere das Fosmidomycinclindamycinsalz und die Salze anderer Lincosamide, zu verstehen.

Besonderheiten der obigen Definitionen und geeignete Beispiele dafür werden nachfolgend angegeben:
"Acyl" ist ein Substituent, der von einer Säure stammt, wie von einer organischen Carbonsäure, Kohlensäure, Carbaminsäure oder der den einzelnen vorstehenden Säuren entsprechenden Thiosäure oder Imidsäure, oder von einer organischen Sulfonsäure, wobei diese Säuren jeweils aliphatische, aromatische und/oder heterocyclische Gruppen im Molekül umfassen sowie Carbamoyl oder Carbamimidoyl.

Geeignete Beispiele für-diese Acylgruppen werden nachfolgend angegeben.

Als aliphatische Acylgruppen werden von einer aliphatischen Säure stammende Acylreste bezeichnet, zu denen die folgenden gehören:
Alkanoyl (z.B. Formyl, Acetyl, Propionyl, Butyryl, Isobutyryl, Valeryl, Isovaleryl, Pivaloyl etc.);
Alkenoyl (z. B. Acryloyl, Methacryloyl, Crotonoyl etc.); Alkylthioalkanoyl (z.B. Methylthioacetyl, Ethylthioacetyl etc.)
Alkansulfonyl (z.B. Mesyl, Ethansulfonyl, Propansulfonyl etc.);
Alkoxycarbonyl (z.B. Methoxycarbonyl, Ethoxycarbonyl, Propoxycarbonyl, Isopropoxycarbonyl, Butoxycarbonyl, Isobutoxycarbonyl etc.);
Alkylcarbamoyl (z.B. Methylcarbamoyl etc.);
(N-Alkyl)-thiocarbamoyl ( z.B. (N-Methyl)-thiocarbamoyl etc.); Alkylcarbamimidoyl (z.B. Methylcarbamimidoyl etc.); Oxalo;
Alkoxalyl (z.B. Methoxalyl, Ethoxalyl, Propoxalyl etc.).

Bei den obigen Beispielen für aliphatische Acylgruppen kann der aliphatische Kohlenwasserstoffteil, insbesondere die Alkylgruppe bzw. der Alkanrest, ggf. einen oder mehrere geeignete Substituenten aufweisen, wie Amino, Halogen (z.B. Fluor, Chlor, Brom etc.), Hydroxy, Hydroxyimino, Carboxy, Alkoxy (z.B. Methoxy, Ethoxy, Propoxy etc.), Alkoxycarbonyl, Acylamino (z.B. Benzyloxycarbonylamino etc.), Acyloxy (z.B. Acetoxy, Benzoyloxy etc.) und dergleichen; als bevorzugte aliphatische Acylreste mit solchen Substituenten sind z.B. mit Amino, Carboxy, Amino und Carboxy, Halogen, Acylamino oder dergleichen substituierte Alkanoyle zu nennen.

Als aromatische Acylreste werden solche Acylreste bezeichnet, die von einer Säure mit substituierter oder nicht substituierter Arylgruppe stammen, wobei die Arylgruppe Phenyl, Tolyl, Xylyl, Naphthyl und dergleichen umfassen kann; geeignete Beispiele werden nachfolgend angegeben:
Aroyl (z.B. Benzoyl, Toluoyl, Xyloyl, Naphthoyl, Phthaloyl etc.);
Aralkanoyl (z.B. Phenylacetyl etc.);
Aralkenoyl (z.B. Cinnamoyl etc.);
Aryloxyalkanoyl (z.B. Phenoxyacetyl etc.);
Arylthioalkanoyl (z.B. Phenylthioacetyl etc.); Arylaminoalkanoyl (z.B. N-Phenylglycyl, etc.);
Arensulfonyl (z.B.Benzolsulfonyl, Tosyl bzw. Toluolsulfonyl, Naphthalirisulfonyl etc.);
Aryloxycarbonyl (z.B. Phenoxycarbonyl, Naphthyl-oxycarbonyl etc.);
Aralkoxycarbonyl (z.B. Benzyloxycarbonyl etc.);
Arylcarbamoyl (z.B. Phenylcarbamoyl, Naphthylcarbamoyl etc.);
Arylglyoxyloyl (z.B. Phenylglyoxyloyl etc.).

Bei den vorstehenden Beispielen für aromatische Acylreste kann der aromatische Kohlenwasserstoffteil (insbesondere der Arylrest) und/oder der aliphatische Kohlenwasserstoffteil (insbesondere der Alkanrest) ggf. einen oder mehrere geeignete Substituenten aufweisen, wie solche, die als geeignete Substituenten für die Alkylgruppe bzw. den Alkanrest bereits angegeben wurden. Insbesondere und als Beispiel für bevorzugte aromatische Acylreste mit besonderen Substituenten werden mit Halogen und Hydroxy oder mit Halogen und Acyloxy substituiertes Aroyl und mit Hydroxy, Hydroxyimino, Dihalogenalkanoyloxyimino substituiertes Aralkanoyl angegeben sowie
Arylthiocarbamoyl (z.B. Phenylthiocarbamoyl etc.); Arylcarbamimidoyl (z.B. Phenylcarbamimidoyl etc.).

Als heterocyclischer Acylrest wird ein Acylrest verstanden, der von einer Säure mit heterocyclischer Gruppe stammt; dazu gehören:

Heterocyclisches Carbonyl, bei dem der heterocyclische Rest ein aromatischer oder aliphatischer 5-bis 6-gliedriger Heterocyclus mit zumindest einem Heteroatom aus der Gruppe Stickstoff, Sauerstoff und Schwefel ist (z.B. Thiophenyl, Furoyl, Pyrrolcarbonyl, Nicotinoyl etc.);

Heterocyclus-Alkanoyl, bei dem der heterocyclische Rest 5- bis 6-gliedrig ist und zumindest ein Heteroatom aus der Gruppe Stickstoff, Sauerstoff und Schwefel aufweist (z.B. Thiophenyl-acetyl, Furylacetyl, Imidazolylpropionyl, Tetrazolylacetyl, 2-(2-Amino-4-thiazolyl)-2-methoxyiminoacetyl etc.) und dergleichen.

Bei den obigen Beispielen für heterocyclische Acylreste kann der Heterocyclus und/oder der aliphatische Kohlenwasserstoffteil ggf. einen oder mehrere geeignete Substituenten aufweisen, wie die gleichen, die als geeignet für Alkyl- und Alkangruppen angegeben wurden.

"Alkylgruppen" sind gerad- oder verzweigtkettiger Alkylreste mit 1 bis 24 Kohlenstoffatomen, wie Methyl, Ethyl, Propyl, Isopropyl, Butyl, Isobutyl, tert.-Butyl, Pentyl, Hexyl und dergleichen. Sie können zum Beispiel mit Hydroxy-, Halogen oder Oxygruppen substituiert sein.

Cycloalkyl steht vorzugsweise für ein ggfs. substituiertes C₃₋₈ -Cycloalkyl; als mögliche Substituenten sind u.a. Alkoxy (z.B. Methoxy, Ethoxy etc.), Halogen (z.B. Fluor, Chlor, Brom etc.), Nitro und dergleichen geeignet.

Aryl ist ein aromatischer Kohlenwasserstoffrest, wie Phenyl Naphthyl usw., der ggf. einen oder mehrere geeignete Substituenten aufweisen kann wie Alkyl, Alkoxy (z.B. Methoxy, Ethoxy etc.), Trifluormethylen, Halogen (z.B. Fluor, Chlor, Brom etc.), Nitro und dergleichen.

Zu "Aralkyl" gehören Mono-, Di-, Triphenylalkyle wie Benzyl, Phenethyl, Benzhydryl, Trityl und dergleichen, wobei der aromatische Teil ggf. ein oder mehrere geeignete Substituenten aufweisen kann wie Alkoxy (z.B. Methoxy, Ethoxy etc.), Halogen (z.B. Fluor, Chlor, Brom etc.), Nitro und dergleichen.

Beim obigen Ester kann der Alkan- und/oder Arenteil wahlweise zumindest einen geeigneten Substituenten aufweisen wie Halogen, Alkoxy, Hydroxy, Nitro oder dergleichen.

Die Erfindung betrifft ferner die Verwendung von 3-N-Formylhydroxylaminoprophylphosphonsäurederivaten oder 3-N-Acetylhydroxylaminoprophylphosphonsäurederivaten in Kombinatin mit Clindamycin, Lincomycin, Pirlimycin und andere Lincosamide, Azithromycin, Erythromycin, Spiramycin, Josamycin, Roxithromycin, Clarithromycin, Midecamycin Lumefantrin, Tafenoquin (WR238,605), Pyronaridin, Dihydroartemisinin, Artemether, Arteether und Artesunat, zur Herstellung eines Medikaments zur therapeutischen und prophylaktischen Behandlung von Infektionen, die durch Bakterien, Protozoen oder mehrzellige Parasiten hervorgerufen werden.

Die Anwendung einer Kombinationstherapie mit Hilfe der pharmazeutischen Präparate der vorliegenden Erfindung bietet den Vorteil der synergistischen Verstärkung der antiparasitären Wirksamkeit der Einzelsubstanzen. Dadurch ergibt sich ferner die Möglichkeit der Reduktion der Dosen und damit der Toxizitäten der Einzelsubstanzen bei gleichzeitiger Erhaltung der antiparasitären Wirksamkeit bei Kombination der Einzelsubstanzen. Eine Kombinationstherapie der oben genannten Einzeltherapieprinzipien bietet ferner die Möglichkeit der Überwindung von Resistenzen.

Bei der Anwendung der Kombinationstherapie ist es möglich, die Wirkstoffe in einer sogenannten fixen Kombination, d. h. in einer einzigen pharmazeutischen Formulierung zu verabreichen, in der beide Wirkstoffe enthalten sind, oder eine sogenannte freie Kombination zu wählen, bei der die Wirkstoffe in Form von getrennten pharmazeutischen Formulierungen gleichzeitig aber auch nacheinander appliziert werden können.

Sind die Wirkstoffe Feststoffe, so können die Wirkstoffe nach üblichen Verfahren zu festen Arzneimittelpräparaten verabreicht werden, in dem man z. B. beide Wirkstoffe miteinander vermischt und mit üblichen Träger- oder Hilfsstoffen beispielsweise zu Tabletten verpreßt. Es ist aber auch möglich, die Wirkstoffe getrennt voneinander in einer verkaufsfertigen Verpackungseinheit zur Verfügung zu stellen, wobei die Verpakkungseinheit die beiden Wirkstoffe in getrennte pharmazeutischen Formulierungen hält.

Die pharmazeutischen Präparate können in flüssiger oder fester Form zur enteralen oder parenteralen Applikation. Hierbei kommen alle üblichen Applikationsformen in Frage, beispielsweise Tabletten, Kapseln, Dragees, Sirupe, Lösungen, Suspensionen. Als Injektionsmedium kommt vorzugsweise Wasser zur Anwendung, welches die bei Injektionslösungen üblichen Zusätze wie Stabilisierungsmittel, Lösungsvermittler und Puffer enthält. Für orale Applikation geeignete Zubereitungen können auf Wunsch Geschmacks- oder Süßstoffe erhalten.

Das folgende Beispiel belegt die positive Wirkung einiger repräsentativer Kombinationspräparate.

### Beispiel

Die Sensitivität von Plasmodium falciparum gegen Fosmidomyicn in Kombination mit verschiedenen Substanzen wurde in einem halbautomatischen Versuchssystem durch Incorporation von [³H]-Hypoxanthin in die DNA der Parasiten bestimmt. Dazu wurden auf Mikrotiterplatten die IC50-Werte von Fosmidomycin und dem jeweiligen Kombinationspartner einzeln und in verschiedenen Mischungsverhältnissen bestimmt. Die Ergebnisse wurden als Summe der fraktionalen inhibitorischen Konzentration (sum fractional inhibitory concentration, FIC) ausgedrückt: $sum FIC = IC ⁢ 50 von Fosmidomycin in Mischung / IC ⁢ 50 von Fosmidomycin allein + IC 50 des Kombinationspartners in Mischung / IC 50 des Kombinationspartners allein$

Sum FIC-Werte < 1 bedeuten Synergismus, Werte > 1 Antargonismus und Werte gleich 1 Addition. Dabei ist zu beachten, daß auch schwach antargonistische Kombinationen therapeutisch wertvoll sein können (sum FIC <2), da beide Medikamente nicht mit der vollen zur Monotherapie nötigen Dosis gegeben werden müssen. In diesem Fall besteht der vorteilhafte Effekt in der besonders schnellen Abtötung der Parasiten und der Resistenzvermeidung.

Folgende sum FIC-Werte wurden mit den Plasmodium falciparum-Stämmen Dd2, 3D7 und HB3 gemessen:

| **Medikament** | **P. falciparum-Stamm** | **sum FIC** |
|---|---|---|
| | Dd2 | 0,42 |
| Clindamycin | 3D7 | 0,40 |
| | HB3 | 0,37 |
| | Dd2 | 0,86 |
| Azithromycin | 3D7 | 0,74 |
| | HB3 | 0,85 |
| | Dd2 | 1,20 |
| Lumefantrin | HB3 | 1,08 |
| | 3D7 | 1,21 |

## Patentansprüche

1. Pharmazeutisches Präparat, enthaltend als Wirkstoff eine Verbindung der allgemeinen Formel (1) 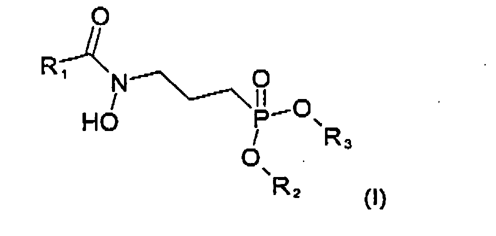 in der R₁ aus der Gruppe ausgewählt ist, die aus Wasserstoff und Methyl besteht,
und
in der R₂ und R₃ unabhängig voneinander aus der Gruppe ausgewählt sind, die aus Wasserstoff, substituiertem oder unsubstituiertem Alkyl, substituiertem oder unsubstituiertem Acyl, substituiertem oder unsubstituiertem Aryl, substituiertem oder unsubstituiertem Aralkyl, substituiertem oder unsubstituiertem Cycloalkyl, einem substituiertem oder unsubstituiertem Silyl, substituiertem oder unsubstituiertem heterocyclischen Rest besteht, oder zusammen eine substituierte oder unsubstituierte C₁₋₅-Alkylkette bilden, wobei die Alkylgruppen gesättigt
oder eine oder mehrere Doppelbindungen oder Dreifachbindungen enthalten können,
in Kombination mit einem zweiten pharmazeutischen Mittel, das aus der Gruppe ausgewählt ist, die aus
Clindamycin, Lincomycin, Pirlimycin und andere Lincosamide,
Azithromycin, Erythromycin, Spiramycin, Josamycin, Roxithromycin, Clarithromycin, Midecamycin,
Lumefantrin, Tafenoquin (WR 238,605), Pyronaridin, Dihydroartemisinin, Artemether, Arteether, Artesunat besteht.

2. Pharmazeutisches Präparat nach Anspruch 1,
**dadurch gekennzeichnet,**
**dass** das zweite pharmazeutischen Mittel aus der Gruppe ausgewählt ist, die aus Lumefantrin, Tafenoquin (WR 238,605), Pyronaridin, Dihydroartemisinin, Artemether, Arteether, Artesunat besteht.

3. Pharmazeutisches Präparat nach Anspruch 1,
**dadurch gekennzeichnet,**
**dass** das zweite pharmazeutischen Mittel aus der Gruppe ausgewählt ist, die aus Clindamycin oder Azithromycin besteht.

4. Verwendung von Verbindungen der Formel (I) 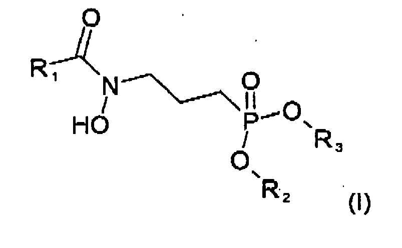 in der R₁ aus der Gruppe ausgewählt ist, die aus Wasserstoff und Methyl besteht,
und
in der R₂ und R₃ unabhängig voneinander aus der Gruppe ausgewählt sind, die aus Wasserstoff, substituiertem oder unsubstituiertem Alkyl, substituiertem oder unsubstituiertem Acyl, substituiertem oder unsubstituiertem Aryl, substituiertem oder unsubstituiertem Aralkyl, substituiertem oder unsubstituiertem Cycloalkyl, einem substituiertem oder unsubstituiertem Silyl, substituiertem oder unsubstituiertem heterocyclischen Rest, besteht oder zusammen eine substituierte oder unsubstituierte C₁₋₅-Alkylkette bilden, wobei die Alkylgruppen gesättigt oder eine oder mehrere Doppelbindungen oder Dreifachbindungen enthalten können,
in Kombination mit einem zweiten pharmazeutischen Mittel, das aus der Gruppe ausgewählt ist, die aus
Clindamycin, Lincomycin, Pirlimycin und andere Lincosamide, Azithromycin, Erythromycin, Spiramycin, Josamycin, Roxithromycin, Clarithromycin, Midecamycin,
Lumefantrin, Tafenoquin (WR 238,605), Pyronaridin, Dihydroartemisinin, Artemether, Arteether, Artesunat besteht, zur Herstellung eines Arzneimittels zur therapeutischen und prophylaktischen Behandlung von Infektionen, die durch Bakterien, Protozoen oder mehrzelligen Parasiten hervorgerufen werden.

5. Verwendung nach Anspruch 4 zur Herstellung eines Arzneimittels zur Behandlung von Malaria.

6. Verwendung nach Anspruch 4,
**dadurch gekennzeichnet,**
**dass** das zweite pharmazeutische Mittel Clindamycin oder Azithromycin ist.

7. Verwendung nach Anspruch 6 zur Herstellung eines Arzneimittels zur Behandlung von Infektionen durch Helicobacter pylori.

## Claims

1. Pharmaceutical preparation comprising as active agent a compound of the general formula (I) 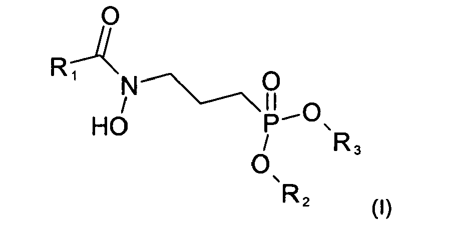 wherein R₁ is selected from the group consisting of hydrogen and methyl,
and
wherein R₂ and R₃ are independently of each other selected from the group consisting of hydrogen, substituted or unsubstituted alkyl, substituted or unsubstituted acyl, substituted or unsubstituted aryl, substituted or unsubstituted aralkyl, substituted or unsubstituted cycloalkyl, a substituted or unsubstituted silyl, substituted or unsubstituted heterocyclic radical, or jointly form a substituted or unsubstituted C₁₋₅-alkylchain, wherein the alkyl groups are saturated or contain one or more double bonds or triple bonds,
in combination with a second pharmaceutical agent selected from the group consisting of
clindamycin, lincomycin, pirlimycin and other lincosamides,
azithromycin, erythromycin, spiramycin, josamycin, roxithromycin, clarithromycin, midecamycin,
lumefantrine, tafenoquine (WR 238,605), pyronaridine, dihydroartemisinin, artemether, arteether, artesunate.

2. Pharmaceutical preparation according to claim 1,
**characterized in that**,
the second pharmaceutical agent is selected from the group consisting of lumefantrine, tafenoquine (WR 238,605), pyronaridine, dihydroartemisinin, artemether, arteether, artesunate.

3. Pharmaceutical preparation according to claim 1,
**characterized in that**,
the second pharmaceutical agent is selected from the group consisting of clindamycin or azithromycin.

4. Use of compounds of the formula (I) 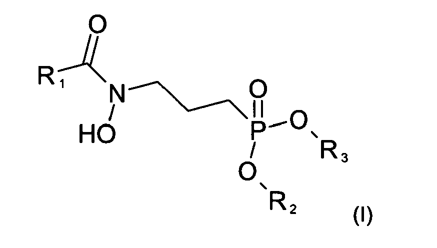 wherein R₁ is selected from the group consisting of hydrogen and methyl,
and
wherein R₂ and R₃ are independently of each other selected from the group consisting of hydrogen, substituted or unsubstituted alkyl, substituted or unsubstituted acyl, substituted or unsubstituted aryl, substituted or unsubstituted aralkyl, substituted or substituted cycloalkyl, a substituted or unsubstituted silyl, substituted or unsubstituted heterocyclic radical, or jointly form a substituted or unsubstituted C₁₋₅-alkylchain, wherein the alkyl groups are saturated or contain one or more double bonds or triple bonds,
in combination with a second pharmaceutical agent selected from the group consisting of
clindamycin, lincomycin, pirlimycin and other lincosamides,
azithromycin, erythromycin, spiramycin, josamycin, roxithromycin, clarithromycin, midecamycin,
lumefantrine, tafenoquine (WR 238,605), pyronaridine, dihydroartemisinin, artemether, arteether, artesunate
for the preparation of a drug for the therapy or prophylaxis of infections, which are caused by bacteria, protozoa or multi-cellular parasites.

5. Use of claim 4 for the preparation of a drug for the treatment of malaria.

6. Use of claim 4,
**characterized in that**,
that the second pharmaceutical agent is clindamycin or azithromycin.

7. Use of claim 6 for the preparation of a drug for the treatment of Infections caused by *Helicobacter pylori.*

## Revendications

1. Une préparation pharmaceutique contient en tant qu'agent un liaison de formule générale (I) 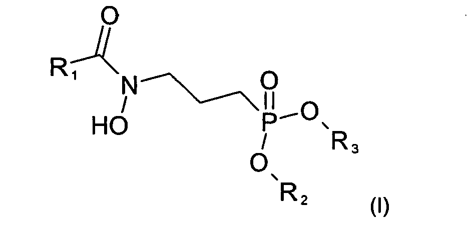 dans laquelle R₁ est choisi dans le groupe comprenant l'hydrogène et méthyle,
et
dans laquelle R₂ et R₃ sont choisis indépendamment dans le groupe comprenant l'hydrogène, alkyle substitué ou non substitué, acyle substitué ou non substitué, aryle substitué ou non substitué, aralkyle substitué ou non substitute, cycloalkyle substitué ou non substitué, un silyle substitué ou non substitué, reste hétérocyclique substitué ou substitué, ou constitue ensemble une C₁₋₅-chaine d'alkyle substitué ou non substitué, dans laquelle les groupes d'alkyle sont saturés ou contient un ou plusieurs liaisons doubles ou liaisons triples,
en combinaison avec une seconde agent pharmaceutique choisi dans le groupe comprenant
clindamycin, lincomycin, pirlimycin et autres lincosamides,
azithromycin, erythromycin, spiramycin, josamycin, roxithromycin, clarithromycin, midecamycin,
lumefantrine, tafenoquine (WR 238,605), pyronaridine, dihydroartemisinine, artemether, arteether, artesunate.

2. Une préparation pharmaceutique selon la revendication 1,
**caractérisée en ce que**,
la seconde agent pharmaceutique est choisi dans le groupe comprenant lumefantrine, tafenoquine (WR 238,605), pyronaridine, dihydroartemisinine, artemether, arteether, artesunate.

3. Une préparation pharmaceutique selon la revendication 1,
**caractérisée en ce que**,
la seconde agent pharmaceutique est choisi dans le groupe comprenant clindamycin ou azithromycin.

4. Utilisation de liaisons de formule (I) 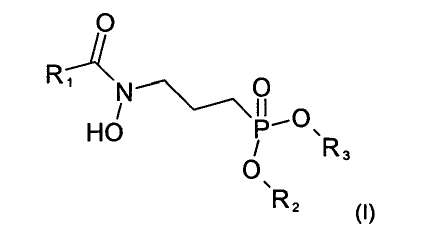 dans laquelle R₁ est choisi dans le groupe comprenant l'hydrogène et méthyle, et
dans laquelle R₂ et R₃ sont choisis indépendamment dans le groupe comprenant l'hydrogène, alkyle substitué ou non substitué, acyle substitué ou non substitué, aryle substitué ou non substitué, aralkyle substitué ou non substitute, cycloalkyle substitué ou non substitué, un silyle substitué ou non substitué, reste hétérocyclique substitué ou substitué, ou forme ensemble une C₁₋₅-chaine d'alkyle substitué ou non substitué, dans laquelle les groupes d'alkyle sont saturés ou contient un ou plusieurs liaisons doubles ou liaisons triples,
en combinaison avec une seconde agent pharmaceutique choisi dans le groupe comprenant
clindamycin, lincomycin, pirlimycin et autres lincosamides,
azithromycin, erythromycin, spiramycin, josamycin, roxithromycin, clarithromycin, midecamycin,
lumefantrine, tafenoquine (WR 238,605), pyronaridine, dihydroartemisinine, artemether, arteether, artesunate
pour la fabrication d'un médicament pour la thérapie ou prophylaxie d'infections, causées par des bactéries, protozoaires ou multicellulaires parasites.

5. Utilisation selon la revendication 4 pour la fabrication d'un médicament pour le traitement de malaria.

6. Utilisation selon la revendication 4,
**caractérisée en ce que**,
la seconde agent pharmaceutique est clindamycin ou azithromycin.

7. Utilisation selon la revendication 6 pour la fabrication d'un médicament pour le traitement d'infections causées par *Helicobacter pylori.*
